# EUROPEAN PATENT SPECIFICATION

(11) **EP 4 578 486 B1**
(45) Date of publication and mention of the grant of the patent: **05.08.2026**
(21) Application number: 24798205.1
(22) Date of filing: 07.03.2024
(51) Int. Cl.: A61N 5/10

(54) **BETA-RAY THERAPY APPARATUS**
BETASTRAHLENTHERAPIEVORRICHTUNG
APPAREIL DE THÉRAPIE PAR RAYONS BETA

(30) Priority: 31.10.2023 CN 202311434045
(43) Date of publication of application: 02.07.2025
(73) Proprietor: Peking Union Medical College Hospital, Chinese Academy of Medical Sciences, Dongcheng District Beijing 100730 (CN)
(72) Inventor: ZHU, Zhaohui, Beijing 100730 (CN); WANG, Jiarou, Beijing 100730 (CN); LI, Linlin, Beijing 100730 (CN); WANG, Rongxi, Beijing 100730 (CN)
(74) Representative: Patrade A/S
(86) International application number: PCT/CN2024/080421
(87) International publication number: WO 2025/091734

(56) References cited:
- CN-A- 110 732 095
- CN-A- 115 103 707
- CN-A- 115 920 253
- CN-A- 117 258 163
- CN-U- 217 612 542
- US-A- 6 096 066
- US-A1- 2016 184 605

## Description

### TECHNICAL FIELD

The present invention relates to the field of external beam radiotherapy of nuclear medicine, and in particular to a β-ray therapy apparatus.

### BACKGROUND

β-rays emitted by radionuclides can be used to effectively treat a variety of skin diseases, and especially have a good therapeutic effect in skin hemangioma and skin keloid. The β-ray applicator should be attached to the lesion surface during treatment.

At present, there are two types of β-ray applicators in common use: one type is a low-dose ³²P applicator, which can be taken home by patients after being attached, resulting in misalignment, falling off, lack of protection, loss, environmental pollution, and other problems. For example, an applicator based on radionuclide ³²P is provided in Chinese patent No. CN212416081 U, and includes a bearing body, and a supporting base capable of being buckled on the bearing body, and a bearing layer for bearing drugs is arranged at the top of the bearing body, and the bearing body can be attached to a diseased area. This scheme solves the problem that the existing ³²P applicator has no radiation protection function and low fitting degree. However, in order to achieve the above purpose, the sealing layer, the bearing layer, a substrate layer and the supporting base are all 3D (three-dimensionally) printed, which are printed after 3D scanning and data analysis of the lesion morphology. Therefore, this scheme needs to be made separately according to the difference of each lesion area, and does not have universal adjustability.

The other is a ⁹⁰Sr-⁹⁰Y applicator with fixed shape and dose, which cannot give different doses of β-ray therapy according to the shape, thickness, and state of the lesion. For example, a ⁹⁰strontium applicator is provided in Chinese application publication No. CN102049098 A, and has a handle, and a protective screen. The handle is provided with a groove in the middle, and an applicator at the lower end. The protective screen is provided with a retaining bead, and a spring. The protective screen is sleeved with the groove at the lower end of the handle, and the retaining bead can fix the protective screen. The purpose of this scheme is that the protective screen can be mounted and dismounted, which is convenient for operation and shortens the radiation exposure time, but in fact, the problem that the applicator cannot be adjusted in shape is not changed.

In conclusion, the existing applicator is not conformable, poor in conformability, or needs to be conformable by 3D printing, and the same apparatus cannot adapt to different lesion shapes, thicknesses, states and the like, which is not universal and adjustable.

US 2016/184605 A1 proposes a robotic applicator device to be deployed internally to a patient having a capsule and aperture with a means of alternately occluding and exposing a radioactive source through the aperture. The capsule and aperture is integrated with a surgical robot to create a robotic IORT (intra-operative radiation therapy) applicator device as more fully described below. The capsule, radiation source, and IORT applicator arm is integrated to enable a physician, physicist or technician to interactively internally view and select tissue for exposure to ionizing radiation in sufficient quantities to deliver therapeutic radiation doses to tissue. Via the robotic manipulation device, the physician and physicist can remotely apply radiation to not only the tissue to be exposed, but also control the length of time of the exposure.

CN 115 920 253 A relates to a use method of a radionuclide applicator, which comprises the following steps: step 1, preparing a 3D scanning device, a 3D printing material, a data processing device and a 3D printing device; performing 3D scanning on the lesion skin of the patient by using a 3D scanning device to obtain lesion form and boundary accurate lesion surface 3D information; 2, lesion surface 3D information data are imported into a data processing device, the data processing device obtains data of an applicator auxiliary positioning device according to the specification of the applicator and the area of the lesion skin, and the applicator auxiliary positioning device comprises a shielding frame and a shielding block; 3, data of the applicator auxiliary positioning device are imported into a 3D printing device, and a 3D printing material is used for printing to obtain an applicator positioning device; and 4, placing the shielding frame and the splicing blocks above the diseased skin of the patient, fixing the positioning device by using an adhesive tape, and fixing the applicator by taking down the splicing blocks in sequence to carry out radioactive therapy.

### SUMMARY

An objective of the present invention is to provide a β-ray therapy apparatus, so as to solve the problems in the prior art. By moving conformable sheets on a conformable frame to different positions, conformable areas with different shapes can be formed according to sites to be treated with different shapes, and then the sites to be treated can be irradiated by β-rays emitted by a β-ray source through the conformable areas. The same therapy apparatus can be used to match with the sites to be treated with different shapes, and has universality and adjustability.

To achieve the objective above, the present invention employs the following technical solution:

The present invention provides a β-ray therapy apparatus, including a therapy apparatus body, and a conformable assembly. The therapy apparatus body includes a β-ray source, and a β-ray shielding assembly. The β-ray shielding assembly is provided with a mounting chamber, and a window communicating with the mounting chamber. The β-ray source is mounted in the mounting chamber. The conformable assembly includes a conformable frame, and multiple conformable sheets, a transmission area is formed in the middle of the conformable frame, the conformable sheets are arranged on the conformable frame, and can move towards or away from the transmission area. A conformable area surrounding a site to be treated is formed in the transmission area by adjusting positions of the conformable sheets. The β-ray source emits β-rays towards the conformable area through the window.

The conformable frame is an annular frame, a hollow region of the annular frame is the transmission area, multiple conformable sheets are divided into two groups, and the two groups of conformable sheets are slidingly arranged on two opposite sides of the annular frame, respectively.

The periphery of the β-ray shielding assembly is provided with multiple first binding ring buckles, the periphery of the conformable frame is provided with multiple second binding ring buckles, and the first binding ring buckles and the second binding ring buckles are respectively configured for connecting with elastic straps.

In some embodiments, the β-ray therapy apparatus includes a β-ray irradiation window. The β-ray irradiation window is mounted at the window, and includes one or more blocking sheets. The blocking sheets move with respect to the window to change a size and shape of the window for adjusting the irradiation range of the β-rays emitted by the β-ray source.

In some embodiments, the blocking sheet is arranged on the β-ray shielding assembly in a penetrating manner, and moves towards or away from the window to shield or open the window.

In some embodiments, when multiple blocking sheets are provided, the multiple blocking sheets are divided into two groups, and the two groups of blocking sheets are slidingly arranged on two opposite sides of the β-ray shielding assembly, respectively.

In some embodiments, two groups of blocking sheets are arranged in one-to-one correspondence to form a plurality of pairs of blocking sheets. In each pair of blocking sheets, one is provided with a V-shaped protrusion, and the other is provided with a V-shaped groove in fit with the V-shaped protrusion. The V-shaped protrusion and the V-shaped groove extend in a width direction of the window.

In some embodiments, the β-ray therapy apparatus includes a controller. The controller is mounted on an outer side of the β-ray shielding assembly, and includes a display screen, an operation button, a dose measuring assembly, and an alerting or alarming assembly.

In some embodiments, the β-ray therapy apparatus includes an auxiliary device. The auxiliary device includes a supporting base, a host, and a mechanical arm. The supporting base is provided with a groove for placing the therapy apparatus body, the host is fixedly mounted on the mechanical arm, the host is provided with a closed storage area, and the mechanical arm is provided with a clamp for clamping the therapy apparatus body.

The present disclosure further provides a method of using the β-ray therapy apparatus above. The method is only illustrative and not part of the claimed invention. The method includes the following steps:
placing a conformable frame at a site to be treated, adjusting positions of conformable sheets according to a shape of the site to be treated, thus forming a conformable area surrounding the site to be treated;
applying ointment or gel to the site to be treated, and making an area applied with the ointment or gel flush with surfaces of the conformable sheets; and
buckling a therapy apparatus body on the conformable frame, and emitting β-rays by a β-ray source, where the β-rays irradiate to the area applied with the ointment or gel through the conformable area.

The invention is set out in the claims.

Compared with the prior art, the present invention has the following technical effects:
(1) By moving the conformable sheets on a conformable frame to different positions, the conformable areas with different shapes can be formed according to sites to be treated with different shapes, and then the sites to be treated can be irradiated by β-rays emitted by a β-ray source through the conformable areas. The same therapy apparatus can be used to match with the sites to be treated with different shapes, and has universality and adjustability.
(2) A β-ray irradiation window is provided, and the size and shape of the window can be changed by moving the blocking sheet, thus adjusting the irradiation range of the β-ray. Based on the existing conformable area, two-stage adjustment of the irradiation range of the β-ray can be formed, such that the β-ray can be controlled more accurately to irradiate a site to be treated, the amount of irradiation to a non-pathological location can be reduced, and normal tissues can be better protected.
(3) The periphery of the β-ray shielding assembly is provided with multiple first binding ring buckles, and the periphery of the conformable frame is provided with multiple second binding ring buckles. The first binding ring buckles and the second binding ring buckles are used to fix the therapy apparatus to a human body by the elastic straps, thus achieving twice fixations, increasing binding effect, fixing the therapy apparatus better, and preventing the influence on the irradiation effect caused by the displacement of the therapy apparatus.
(4) After the conformable area is formed by using the conformable sheets, the ointment or gel is applied to the site to be treated, and uneven lesion can be applied with the ointment or gel to the same level, such that the irradiation dose on the site with thick lesion (thin applying thickness) is more, while the irradiation dose on the site with thin lesion (thick applying thickness) is less, thus different degrees of lesions can be irradiated with different intensities to achieve the effect of intensity modulation.

### BRIEF DESCRIPTION OF THE DRAWINGS

To describe the technical solutions of the embodiments of the present invention or in the prior art more clearly, the following briefly introduces the accompanying drawings required for describing the embodiments. Apparently, the accompanying drawings in the following description show merely some embodiments of the present invention, and those of ordinary skill in the art may still derive other drawings from these accompanying drawings without creative efforts.
FIG. 1 is a front cross-sectional view of a therapy apparatus body according to the present invention;
FIG. 2 is a top view of a controller according to the present invention;
FIG. 3 is a top cross-sectional view of a β-ray shielding assembly according to the present invention;
FIG. 4 is a top view of a blocking sheet according to the present invention;
FIG. 5 is a front view of a supporting base according to the present invention;
FIG. 6 is a top view of FIG. 5;
FIG. 7 is a front cross-sectional view of a conformable assembly according to the present invention;
FIG. 8 is a top view of FIG. 7;
FIG. 9 is a schematic diagram of the present invention including an auxiliary device therein;
FIG. 10 is a top view of FIG. 9.

In the drawings: 1-controller; 2-β-ray shielding assembly; 3-β-ray source; 4-blocking sheet; 5-supporting base; 6-conformable frame; 7-conformable sheet; 8-first binding ring buckle; 9-display screen; 10-operation button; 11-second binding ring buckle; 12-clamp; 13-mechanical arm; 14-host; 15-closed storage area.

### DETAILED DESCRIPTION OF THE EMBODIMENTS

The following clearly and completely describes the technical solutions in the embodiments of the present invention with reference to the accompanying drawings in the embodiments of the present invention. Apparently, the described embodiments are merely a part rather than all of the embodiments of the present invention. All other embodiments obtained by a person of ordinary skill in the art based on the embodiments of the present invention without creative efforts shall fall within the protection scope of the present invention.

An objective of the present invention is to provide a β-ray therapy apparatus, so as to solve the problems in the prior art. By moving conformable sheets on a conformable frame to different positions, conformable areas with different shapes can be formed according to sites to be treated with different shapes, and then the sites to be treated can be irradiated by β-rays emitted by a β-ray source through the conformable areas. The same therapy apparatus can be used to match with the sites to be treated with different shapes, and has universality and adjustability.

In order to make the objectives, features and advantages of the present invention more clearly, the present invention is further described in detail below with reference to the accompanying drawings and specific embodiments.

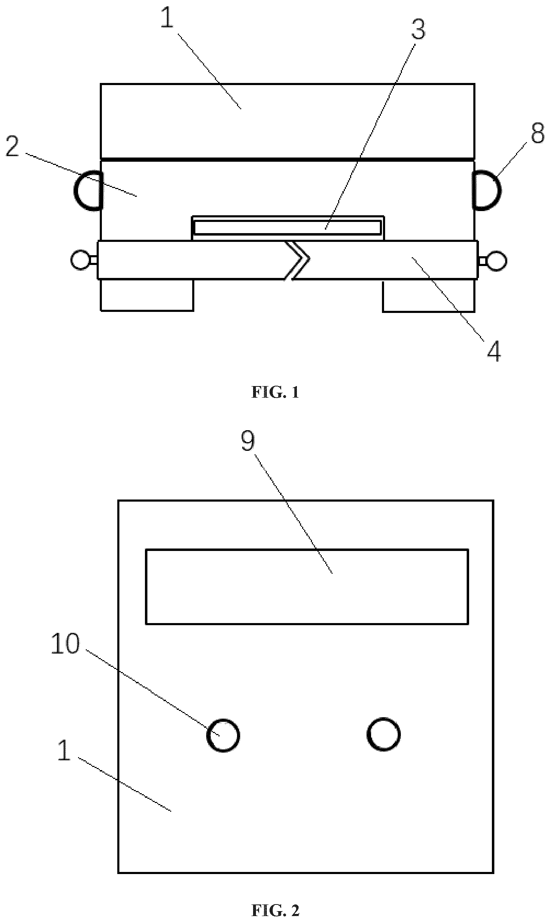

As shown in FIG. 1 to FIG. 8, the present invention provides a β-ray therapy apparatus, including a therapy apparatus body, and a conformable assembly. The therapy apparatus body is mainly used to provide a β-ray for irradiation, and the conformable assembly is used for limiting the irradiation range of the β-ray. Specifically, the therapy apparatus body includes a β-ray source 3, and a β-ray shielding assembly 2. The β-ray shielding assembly 2 is provided with a mounting chamber and a window communicating with the mounting chamber. The β-ray source 3 is mounted in the mounting chamber, which may be a ⁹⁰Sr-⁹⁰Y sealing source, ³²P or other radioactive sources capable of emitting β-ray nuclides. The β-ray shielding assembly 2 may be made of a lightweight material such as aluminum, resin, or plastic, and is used for shielding the β-ray source 3 from emitting rays to other directions except for a lesion direction, thus protecting the safety of the people around. The conformable assembly includes a conformable frame 6 and multiple conformable sheets 7. A transmission area for the β-ray to penetrate is formed in the middle of the conformable frame 6. The conformable sheets 7 are arranged on the conformable frame 6. An end, away from the conformable frame 6, of each conformable sheet 7 is provided with a handle, and the handle can be used to pull or push the conformable sheet 7 to move towards or away from the transmission area, thus adjusting the area and position for blocking the transmission area. When there are multiple conformable sheets 7, by adjusting the lengths of different conformable sheets 7 penetrating into the transmission area (that is, the blocking area and position are different), a conformable area surrounding a site to be treated can be formed in the transmission area by adjusting positions of the conformable sheets 7 according to the shape of the site to be treated, and the shape of the conformable area is basically identical with that of the site to be treated. It should be noted that the more the number and the smaller the size of the conformable sheets 7, the finer the conformable area can be. Here, the specification and size of the conformable sheet 7 are not restricted. In addition, there is no requirement on neither the shape of the β-ray shielding assembly 2 nor the shape of the window, which may be circular, rectangular or other shapes, and the opening range of the window can cover the area of the site to be treated, such that the adjusted conformable area can cover the site to be treated. The β-ray source 3 emits β-rays in the mounting chamber, such that the β-rays can emit β-rays towards the conformable area through the window, thus irradiating and treating the diseased site. In conclusion, by moving conformable sheets 7 on the conformable frame 6 to different positions, the conformable areas with different shapes can be formed according to sites to be treated with different shapes, and then the sites to be treated can be irradiated by β-rays emitted by the β-ray source 3 through the conformable areas. The same therapy apparatus can be used to match with the sites to be treated with different shapes, and has universality and adjustability.

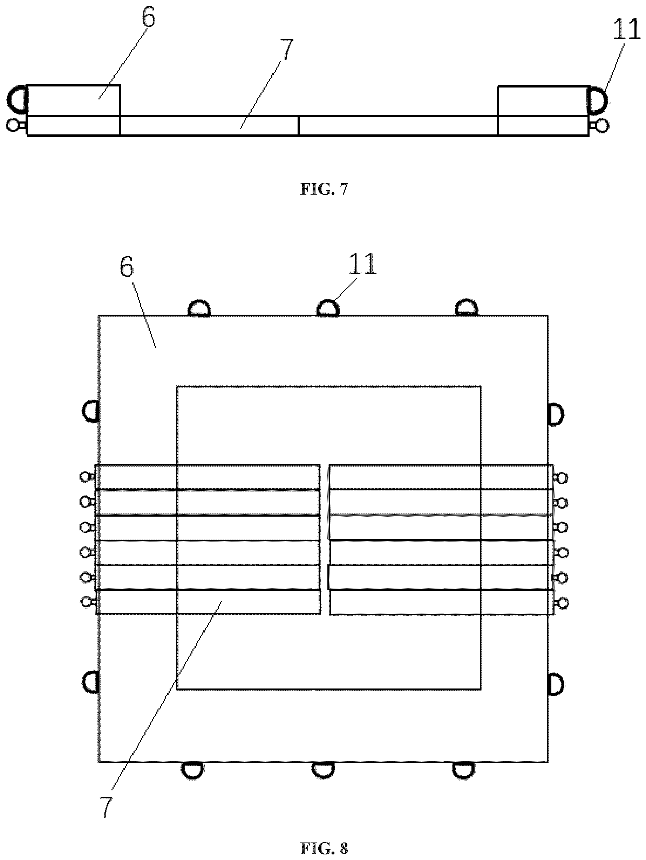

As shown in FIG. 7 and FIG. 8, the conformable frame 6 may be an annular frame, a main body of the annular frame is annular, and the annular shape here may be rectangular, circular or other shapes that can form a ring, not specifically a circular ring. A hollow region of the annular frame is a transmission area, multiple conformable sheets 7 are divided into two groups, and each group includes multiple conformable sheets 7 arranged in parallel. The two groups of conformable sheets 7 are slidingly arranged on two opposite sides of the annular frame, respectively, and the blocked width shape and area of the transmission area can be changed by relatively inserting or pulling out the conformable sheets 7. Further, the conformable frame 6 may be a rectangular frame shown in FIG. 8, while the conformable sheet 7 is a long-striped rectangular sheet, and two groups of rectangular sheets are located on two opposite edges of the rectangular frame, respectively.

As shown in FIG. 1, FIG. 3, FIG. 7 and FIG. 8, the periphery of the β-ray shielding assembly 2 is provided with multiple first binding ring buckles 8, and the periphery of the conformable frame 6 is provided with multiple second binding ring buckles 11. The first binding ring buckles 8 and the second binding ring buckles 11 each are arranged at intervals, and at least two binding ring buckles are arranged on each edge. The first binding ring buckles 8 and the second binding ring buckles 11 are used to connect elastic straps, and the therapy apparatus can be fixed to the human body by the first binding ring buckles 8 and the second binding ring buckles 11 through the elastic straps, thus achieving twice fixations, increasing binding effect, fixing the therapy apparatus better, and preventing the influence on the irradiation effect caused by the displacement of the therapy apparatus.

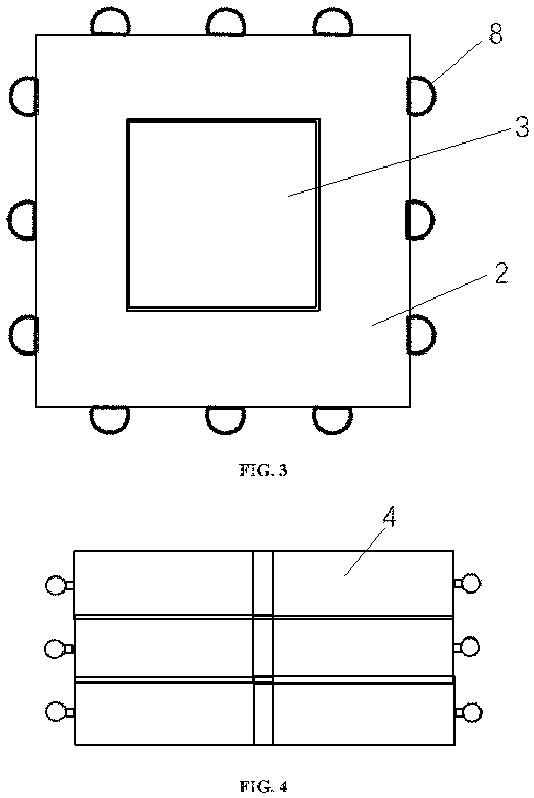

As shown in FIG. 1 and FIG. 4, the β-ray therapy apparatus includes a β-ray irradiation window. The β-ray irradiation window is mounted at the window of the β-ray shielding assembly 2. The β-ray irradiation window includes one or more blocking sheets 4. The blocking sheet 4 is made of a lightweight material such as aluminum, resin, or plastic. The blocking sheet 4 may be directly arranged on the β-ray shielding assembly 2 in a penetrating manner, or arranged on a window structure, and then mounted on the window through the window structure. The blocking sheet 4 can move horizontally or longitudinally when moving with respect to the window, preferably in a longitudinal moving mode of inserting or pulling out. An end, away from the β-ray shielding assembly 2, of the blocking sheet 4 is provided with a handle, and the handle is used to drive the blocking sheet 4 to move. When the therapy apparatus body does not perform irradiation, the window can be completely closed by the blocking sheet 4 to avoid improper emission of β-rays. When only one blocking sheet 4 is provided, the blocking sheet 4 can be used to reciprocate in one direction to change the size of the window. When multiple blocking sheets 4 are provided, the window size can be changed by moving different blocking sheets 4, and the size of the window can be in fit with the shape of the site to be treated at the same time. The change of the window can adjust the irradiation range of the β-rays emitted by the β-ray source 3. More specifically, on the basis of forming the conformable area by using the existing conformable assembly, two-stage adjustment of the irradiation range of the β-ray can be formed, such that the β-ray can be controlled more accurately to irradiate a site to be treated, the amount of irradiation to a non-pathological location can be reduced, and normal tissues can be better protected.

More specifically, the β-ray shielding assembly 2 may be provided with a through hole or slot, the blocking sheet 4 may be arranged in the through hole or slot in a penetrating manner, and can move towards or away from the window to shield or open the window.

When multiple blocking sheets 4 are provided, the multiple blocking sheets 4 are divided into two groups, and each group include multiple blocking sheets 4 arranged in parallel. The two groups of blocking sheets 4 are slidingly arranged on two opposite sides of the β-ray shielding assembly 2, respectively. When the β-ray shielding assembly 2 is a rectangular frame, the blocking sheet 4 is a rectangular sheet, and the rectangular sheets are arranged on two opposite edges of the rectangular frame.

Two groups of blocking sheets 4 are arranged in one-to-one correspondence to form multiple pairs of blocking sheets. In each pair of blocking sheets 4, one blocking sheet is provided with a V-shaped protrusion, the other blocking sheet is provided with a V-shaped groove in fit with the V-shaped protrusion. The V-shaped protrusion and the V-shaped groove extend in a width direction of the window. When the blocking sheets 4 close to each other, the V-shaped protrusions can be inserted into the V-shaped grooves to form up-and-down shielding, thereby reducing a gap, and improving the blocking effect to the β-rays.

As shown in FIG. 1 and FIG. 2, the β-ray therapy apparatus includes a controller 1. The controller 1 is mounted on an outer side of the β-ray shielding assembly 2, and includes a display screen 9, an operation button 10, a dose measuring assembly, and an alerting or alarming assembly. The dose measuring assembly is used to measure the radiation dose received by the diseased site, and the display screen 9 is used to display the measured radiation dose received by the diseased site. When the dose or time to be received is about to reach, an alerting or alarming sound can be given by the alerting or alarming assembly, and the operation of the controller 1 can be controlled by the operation button 10, such as starting or stopping, and setting the dose alerting value.

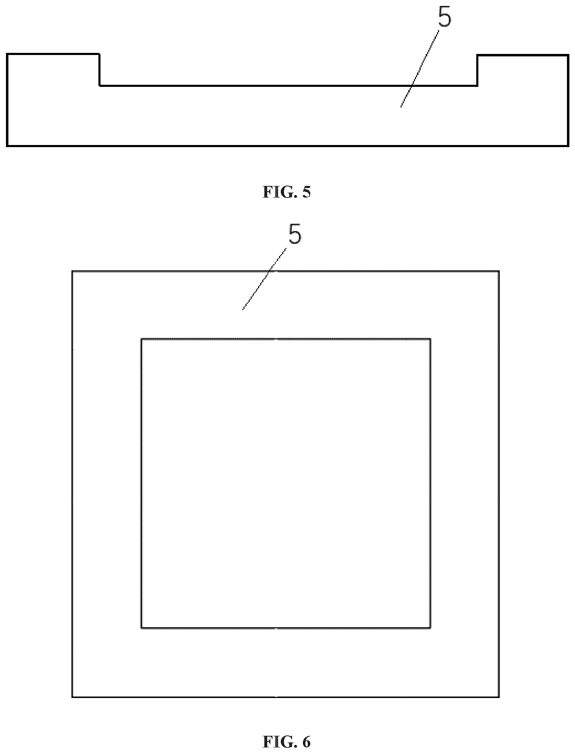

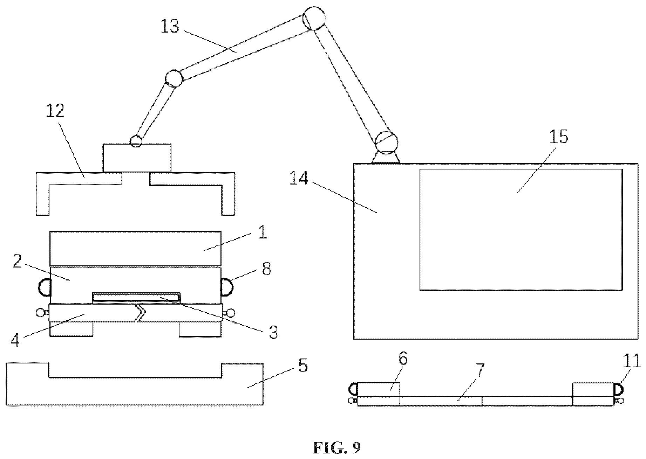

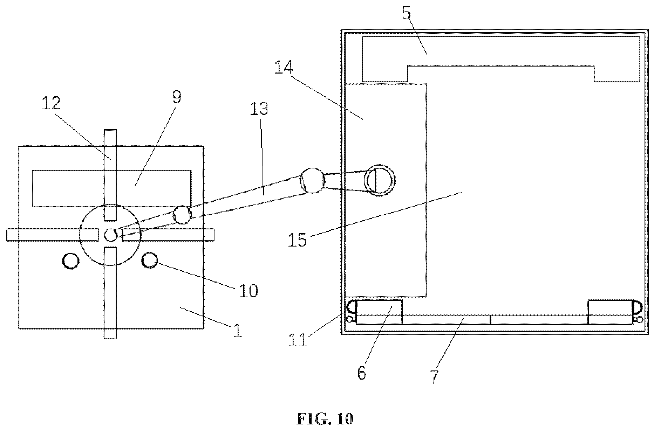

As shown in FIG. 5, FIG. 6, FIG. 9 and FIG. 10, the β-ray therapy apparatus includes an auxiliary device, including a supporting base 5, a host 14, and a mechanical arm 13. The supporting base 5 is provided with a groove for placing the therapy apparatus body. When the therapy apparatus is idle, the therapy apparatus body can be supported by the supporting base. The mechanical arm 13 can be fixedly mounted on the host 14 to support the movement of the mechanical arm 13. Meanwhile, the host 14 may also be provided with a closed storage area 15, which can be used for storing the supporting base 5 and the conformable assembly, as well as the therapy apparatus body. Both the supporting base 5 and the conformable assembly can be placed laterally in the closed storage area 15.

The mechanical arm 13 is provided with a clamp 12 for clamping the therapy apparatus body. Through the arrangement of the mechanical arm 13 and the clamp 12, on the one hand, the therapy apparatus body can be moved to the site to be treated by the mechanical arm 13, and kept at a corresponding position state for irradiation; on the other hand, the therapy apparatus body after use can be returned to the closed storage area 15.

When the apparatus provided by the present invention is used for treating hyperplastic diseases such as scar, hemangioma, tumor, circumscribed scleroderma, etc., β-ray irradiation windows with different opening sizes can be obtained by adjusting the positions of the blocking sheets 4, conformable areas with different opening sizes can be obtained by adjusting the conformable sheets 7, the application shape can be selected according to the shape, thickness and state of the lesion, and the irradiation range of the β-ray source 3 can be adjusted. The therapy apparatus body and the conformable assembly can be fixed to the surface of the human body through the elastic straps, and are not easy to fall off. The clamp 12 and the mechanical arm 13 can be used to fix a treatment position of the therapy apparatus and store the therapy apparatus into the closed storage area 15, thus facilitating use and recovery.

As shown in FIG. 1 to FIG. 10, the present disclosure further provides a method of using the β-ray therapy apparatus described above, including the following steps:

A conformable frame 6 is placed at a site to be treated, and can be fixed by second binding ring buckles 11 and elastic straps. Position of conformable sheets 7 are adjusted according to the shape of the site to be treated, thus forming a conformable area surrounding the site to be treated and achieving the effect of shape adjustment.

Ointment for burns, ointment for scars or gel is applied to the site to be treated, and uneven lesion is applied with the ointment or gel to the same level, making an area applied with the ointment or gel flush with the surfaces of conformable sheets 7, such that the irradiation dose on the site with thick lesion (thin applying thickness) is more, while the irradiation dose on the site with thin lesion (thick applying thickness) is less, thus different degrees of lesions can be irradiated with different intensities to achieve the effect of intensity modulation.

The therapy apparatus body can be buckled on the conformable frame 6, which can be clamped by the mechanical arm 13, or fixed by the first binding ring buckles 8 and the elastic straps. B-rays are emitted by the β-ray source 3, and the β-rays can irradiate the area applied with the ointment or gel through the conformable area.

In order to describe the method of using the present invention better, the following specific embodiments are provided.

If a patient has a scar to be treated on a forearm. The radiation dose needed for treatment is calculated according to the size of the scar and then is set as an alerting threshold of the controller 1. The shape of the β-ray irradiation window is adjusted to be the same as that of the scar by adjusting the position of the blocking sheet 4. The scar site is surrounded by the conformable assembly, then the conformable assembly is fixed by the elastic straps, and the conformable sheets 7 are adjusted to form a conformable area consistent with the shape of the scar. After a plane on the uneven scar site is formed by using the gel, the therapy apparatus body is fixed to the forearm of the patient through the elastic straps to start irradiation treatment. After reaching the irradiation dose required for treatment, the controller 1 gives an alerting sound, then the elastic straps can be loosened, and the therapy apparatus body is stored in the closed storage area 15 by the clamp 12 and the mechanical arm 13.

Specific examples are used herein for illustration of the principles and embodiments of the present invention. In addition, a person of ordinary skill in the art can make various modifications in terms of specific embodiments and scope of application in accordance with the teachings of the present invention.

## Claims

1. A β-ray therapy apparatus, comprising a therapy apparatus body, and a conformable assembly, wherein the therapy apparatus body comprises a β-ray source (3), and a β-ray shielding assembly (2) provided with a mounting chamber and a window communicating with the mounting chamber; the β-ray source (3) is mounted in the mounting chamber; the conformable assembly comprises a conformable frame (6) and a plurality of conformable sheets (7), a transmission area is formed in a middle of the conformable frame (6), the plurality of conformable sheets (7) are arranged on the conformable frame (6), and are able to move towards or away from the transmission area; a conformable area surrounding a site to be treated is formed in the transmission area by adjusting positions of the plurality of conformable sheets (7); and the β-ray source (3) is configured to emit β-rays towards the conformable area through the window;
wherein the conformable frame (6) is an annular frame, a hollow region of the annular frame is the transmission area, the plurality of conformable sheets (7) are divided into two groups, and two groups of the plurality of conformable sheets (7) are slidingly arranged on two opposite sides of the annular frame, respectively; and
**characterized in that** a periphery of the β-ray shielding assembly (2) is provided with a plurality of first binding ring buckles (8), a periphery of the conformable frame (6) is provided with a plurality of second binding ring buckles (11), and the first binding ring buckles (8) and the second binding ring buckles (11) are respectively configured for connecting with elastic straps.

2. The β-ray therapy apparatus according to claim 1, comprising a β-ray irradiation window, wherein the β-ray irradiation window is mounted at the window, and comprises one or more blocking sheets (4); and the blocking sheets (4) are configured to move with respect to the window to change a size and shape of the window for adjusting an irradiation range of the β-rays emitted by the β-ray source (3).

3. The β-ray therapy apparatus according to claim 2, wherein the one or more blocking sheets (4) are arranged on the β-ray shielding assembly (2) in a penetrating manner, and are configured to move towards or away from the window to shield or open the window.

4. The β-ray therapy apparatus according to claim 3, wherein when a plurality of blocking sheets (4) are provided, the plurality of blocking sheets (4) are divided into two groups, and the two groups of blocking sheets (4) are slidingly arranged on two opposite sides of the β-ray shielding assembly (2), respectively.

5. The β-ray therapy apparatus according to claim 4, wherein the two groups of blocking sheets (4) are arranged in one-to-one correspondence to form a plurality of pairs of blocking sheets (4); in each pair of blocking sheets (4), one blocking sheet (4) is provided with a V-shaped protrusion, and an other blocking sheet (4) is provided with a V-shaped groove in fit with the V-shaped protrusion; and the V-shaped protrusion and the V-shaped groove extend in a width direction of the window.

6. The β-ray therapy apparatus according to claim 1, comprising a controller (1), wherein the controller (1) is mounted on an outer side of the β-ray shielding assembly (2), and comprises a display screen (9), an operation button (10), a dose measuring assembly, and an alerting or alarming assembly.

7. The β-ray therapy apparatus according to claim 1, comprising an auxiliary device, wherein the auxiliary device comprises a supporting base (5), a host (14), and a mechanical arm (13); the supporting base (5) is provided with a groove for placing the therapy apparatus body, the host (14) is fixedly mounted on the mechanical arm (13), the host (14) is provided with a closed storage area (15), and the mechanical arm (13) is provided with a clamp (12) for clamping the therapy apparatus body.

## Patentansprüche

1. β-Strahlentherapievorrichtung, umfassend einen Therapievorrichtungskörper und eine anpassbare Baugruppe, wobei der Therapievorrichtungskörper eine β-Strahlenquelle (3) und eine β-Strahlenabschirmungsbaugruppe (2) umfasst, die mit einer Montagekammer und einem mit der Montagekammer kommunizierenden Fenster versehen ist; wobei die β-Strahlenquelle (3) in der Montagekammer montiert ist; wobei die anpassbare Baugruppe einen anpassbaren Rahmen (6) und eine Vielzahl von anpassbaren Platten (7) umfasst, ein Übertragungsbereich in einer Mitte des anpassbaren Rahmens (6) ausgebildet ist, die Vielzahl von anpassbaren Platten (7) auf dem anpassbaren Rahmen (6) angeordnet ist und in der Lage ist, sich in Richtung des Übertragungsbereichs oder von diesem weg zu bewegen; wobei ein anpassbarer Bereich, der eine zu behandelnde Stelle umgibt, durch Einstellen von Positionen der Vielzahl von anpassbaren Platten (7) ausgebildet ist; und wobei die β-Strahlenquelle (3) konfiguriert ist, β-Strahlen durch das Fenster in Richtung des anpassbaren Bereichs abzustrahlen;
wobei der anpassbare Rahmen (6) ein ringförmiger Rahmen ist, eine hohle Region des ringförmigen Rahmens der Übertragungsbereich ist, die Vielzahl von anpassbaren Platten (7) in zwei Gruppen unterteilt ist und zwei Gruppen der Vielzahl von anpassbaren Platten (7) jeweils verschiebbar auf zwei gegenüberliegenden Seiten des ringförmigen Rahmens angeordnet sind; und
**dadurch gekennzeichnet, dass** ein Umfang der β-Strahlenabschirmungsbaugruppe (2) mit einer Vielzahl von ersten Bindungsringschnallen (8) versehen ist, ein Umfang des anpassbaren Rahmens (6) mit einer Vielzahl von zweiten Bindungsringschnallen (11) versehen ist und die ersten Bindungsringschnallen (8) beziehungsweise die zweiten Bindungsringschnallen (11) zum Verbinden mit elastischen Bändern konfiguriert sind.

2. β-Strahlentherapievorrichtung nach Anspruch 1, umfassend ein β-Strahlenbestrahlungsfenster, wobei das β-Strahlenbestrahlungsfenster an dem Fenster montiert ist und eine oder mehrere Blockierplatten (4) umfasst; und wobei die Blockierplatten (4) konfiguriert sind, sich in Bezug auf das Fenster zu bewegen, um eine Größe und Form des Fensters zum Einstellen eines Bestrahlungsfelds der von der β-Strahlenquelle (3) abgestrahlten β-Strahlen zu ändern.

3. β-Strahlentherapievorrichtung nach Anspruch 2, wobei die eine oder mehreren Blockierplatten (4) auf der β-Strahlenabschirmungsbaugruppe (2) auf eine durchdringende Weise angeordnet sind und konfiguriert sind, sich in Richtung des Fensters oder von diesem weg zu bewegen, um das Fenster abzuschirmen oder zu öffnen.

4. β-Strahlentherapievorrichtung nach Anspruch 3, wobei, wenn eine Vielzahl von Blockierplatten (4) bereitgestellt ist, die Vielzahl von Blockierplatten (4) in zwei Gruppen unterteilt ist und die beiden Gruppen von Blockierplatten (4) jeweils verschiebbar auf zwei gegenüberliegenden Seiten der β-Strahlenabschirmungsbaugruppe (2) angeordnet sind.

5. β-Strahlentherapievorrichtung nach Anspruch 4, wobei die beiden Gruppen von Blockierplatten (4) in einer Eins-zu-eins-Entsprechung angeordnet sind, um eine Vielzahl von Paaren von Blockierplatten (4) auszubilden; wobei in jedem Paar von Blockierplatten (4) eine Blockierplatte (4) mit einem V-förmigen Vorsprung versehen ist und eine andere Blockierplatte (4) mit einer V-förmigen Nut versehen ist, die mit dem V-förmigen Vorsprung zusammenpasst; und wobei sich der V-förmige Vorsprung und die V-förmige Nut in eine Breitenrichtung des Fensters erstrecken.

6. β-Strahlentherapievorrichtung nach Anspruch 1, umfassend eine Steuerung (1), wobei die Steuerung (1) an einer Außenseite der β-Strahlenabschirmungsbaugruppe (2) montiert ist und einen Anzeigebildschirm (9), einen Bedienknopf (10), eine Dosismessbaugruppe und eine Warn- oder Alarmbaugruppe umfasst.

7. β-Strahlentherapievorrichtung nach Anspruch 1, umfassend eine Hilfseinrichtung, wobei die Hilfseinrichtung eine Trägerbasis (5), eine Aufnahme (14) und einen mechanischen Arm (13) umfasst; wobei die Trägerbasis (5) mit einer Nut zum Platzieren des Therapievorrichtungskörpers versehen ist, die Aufnahme (14) fest an dem mechanischen Arm (13) montiert ist, die Aufnahme (14) mit einem geschlossenen Stauraumbereich (15) versehen ist und der mechanische Arm (13) mit einer Klemme (12) zum Klemmen des Therapievorrichtungskörpers versehen ist.

## Revendications

1. Appareil de thérapie par rayons β, comprenant un corps d'appareil de thérapie et un ensemble conformable, dans lequel le corps d'appareil de thérapie comprend une source de rayons β (3) et un ensemble de blindage contre les rayons β (2) muni d'une chambre de montage et d'une fenêtre communiquant avec la chambre de montage ; la source de rayons β (3) est montée dans la chambre de montage ; l'ensemble conformable comprend un cadre conformable (6) et une pluralité de feuilles conformables (7), une zone de transmission est formée au milieu du cadre conformable (6), la pluralité de feuilles conformables (7) sont agencées sur le cadre conformable (6) et peuvent se déplacer vers ou à l'écart de la zone de transmission ; une zone conformable entourant un site à traiter est formée dans la zone de transmission en réglant les positions de la pluralité de feuilles conformables (7) ; et la source de rayons β (3) est configurée pour émettre des rayons β vers la zone conformable à travers la fenêtre ;
dans lequel le cadre conformable (6) est un cadre annulaire, une région creuse du cadre annulaire est la zone de transmission, la pluralité de feuilles conformables (7) est divisée en deux groupes, et les deux groupes de la pluralité de feuilles conformables (7) sont agencés de manière coulissante sur deux côtés opposés du cadre annulaire, respectivement ; et **caractérisé en ce qu'**une périphérie de l'ensemble de blindage contre les rayons β (2) est munie d'une pluralité de premières boucles d'anneau de liaison (8), une périphérie du cadre conformable (6) est munie d'une pluralité de secondes boucles d'anneau de liaison (11), et les premières boucles d'anneau de liaison (8) et les secondes boucles d'anneau de liaison (11) sont respectivement configurées pour se raccorder à des sangles élastiques.

2. Appareil de thérapie par rayons β selon la revendication 1, comprenant une fenêtre d'irradiation par rayons β, dans lequel la fenêtre d'irradiation par rayons β est montée au niveau de la fenêtre et comprend une ou plusieurs feuilles de blocage (4) ; et les feuilles de blocage (4) sont configurées pour se déplacer par rapport à la fenêtre afin de modifier une taille et une forme de la fenêtre pour régler une plage d'irradiation des rayons β émis par la source de rayons β (3).

3. Appareil de thérapie par rayons β selon la revendication 2, dans lequel les une ou plusieurs feuilles de blocage (4) sont agencées sur l'ensemble de blindage contre les rayons β (2) de manière pénétrante et sont configurées pour se déplacer vers ou à l'écart de la fenêtre afin de protéger ou d'ouvrir la fenêtre.

4. Appareil de thérapie par rayons β selon la revendication 3, dans lequel, lorsqu'une pluralité de feuilles de blocage (4) sont fournies, la pluralité de feuilles de blocage (4) sont divisées en deux groupes, et les deux groupes de feuilles de blocage (4) sont agencés de manière coulissante sur deux côtés opposés de l'ensemble de blindage contre les rayons β (2), respectivement.

5. Appareil de thérapie par rayons β selon la revendication 4, dans lequel les deux groupes de feuilles de blocage (4) sont agencés en correspondance un à un pour former une pluralité de paires de feuilles de blocage (4) ; dans chaque paire de feuilles de blocage (4), une feuille de blocage (4) est munie d'une saillie en forme de V et une autre feuille de blocage (4) est munie d'une rainure en forme de V s'emboîtant dans la saillie en forme de V ; et la saillie en forme de V et la rainure en forme de V s'étendent dans la direction de la largeur de la fenêtre.

6. Appareil de thérapie par rayons β selon la revendication 1, comprenant un dispositif de commande (1), dans lequel le dispositif de commande (1) est monté sur un côté externe de l'ensemble de blindage contre les rayons β (2), et comprend un écran d'affichage (9), un bouton de fonctionnement (10), un ensemble de mesure de dose et un ensemble d'alerte ou d'alarme.

7. Appareil de thérapie par rayons β selon la revendication 1, comprenant un dispositif auxiliaire, dans lequel le dispositif auxiliaire comprend une base de support (5), un hôte (14) et un bras mécanique (13) ; la base de support (5) est munie d'une rainure pour placer le corps d'appareil de thérapie, l'hôte (14) est monté de manière fixe sur le bras mécanique (13), l'hôte (14) est muni d'une zone de stockage fermée (15) et le bras mécanique (13) est muni d'une pince (12) pour fixer le corps d'appareil de thérapie.
